(19) Europäisches Patentamt
European Patent Office
Office européen des brevets

(11) Publication number: **0 171 760**
**A2**

# (12) EUROPEAN PATENT APPLICATION

(21) Application number: 85109984.6

(22) Date of filing: 08.08.85

(51) Int. Cl.⁴: C 07 C 103/34
A 61 K 31/16

(30) Priority: 16.08.84 GB 8420837

(43) Date of publication of application: 19.02.86 Bulletin 86/8

(84) Designated Contracting States: BE CH DE FR GB IT LI NL

(71) Applicant: BEECHAM GROUP PLC
Beecham House Great West Road
Brentford Middlesex TW8 9BD(GB)

(72) Inventor: Duckworth, David Malcolm
44 Meadow Walk Walton-on-the-Hill
Tadworth Surrey, KT20 7UG(GB)

(72) Inventor: Jennings, Leslie John Arthur
2 Logmore Green Cottage Logmore Lane
Westcott Surrey, RH4 3JN(GB)

(74) Representative: Hardman, Carol Pauline et al,
Beecham Pharmaceuticals Great Burgh Yew Tree Bottom Road
Epsom Surrey KT18 5XQ(GB)

## (54) Substituted phenoxypropylaminoethoxyphenoxyacetamides.

(57) Compounds of the general formula (I)

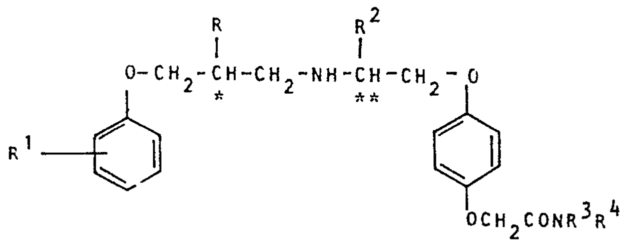

(I)

, or a pharmaceutically acceptable salt thereof, in which
R represents a hydroxy group or an *in vivo* hydrolysable ester thereof,
$R^1$ represents a hydroxy group in the 2-, 3-, or 4-position, or an ester thereof,
$R^2$ represents a hydrogen atom or a lower alkyl group,
$R^3$ and $R^4$, which may be the same or different, each represents a hydrogen atom; an alkyl radical; or an unsubstituted or substituted aryl, arylalkyl or diarylalkyl radical; or
$R^3$ and $R^4$, together with the nitrogen atom to which they are attached, form a 5-, 6- or 7-membered saturated or unsaturated heterocyclic radical; a process for preparing such a compound, pharmaceutical compositions containing them; and their use in medicine.

EP 0 171 760 A2

Croydon Printing Company Ltd

TITLE MODIFIED
see front page

# Novel Compounds

The invention relates to secondary amines which have potent inotropic activity, to processes for their preparation; pharmaceutical compositions containing them and their use in medicine.

EP 0015505 A discloses certain secondary amines having positive inotropic activity, and EP 0023385 B discloses other secondary amines having hypoglycaemic and anti-obesity activity.

We have now discovered a group of pharmacologically active compounds somewhat related to, but structurally distinct from, those of the above-mentioned prior art.

More particularly, the compounds are 3-phenoxypropan-2-olamines also containing a phenoxyacetamide unit.

The compounds have positive inotropic activity: they increase the force of contraction of the heart but produce no or little corresponding increase in heart rate. They may therefore be used for treating congestive heart failure.

Thus, more particularly, the present invention provides a compound of the general formula (I)

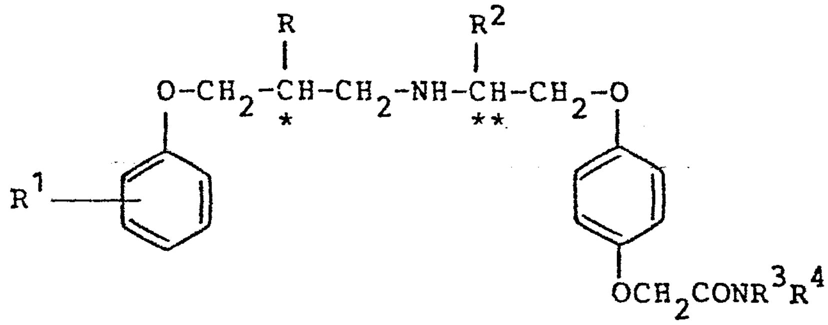

(I)

in which

R represents a hydroxy group or an _in vivo_ hydrolysable ester thereof,

$R^1$ represents a hydroxy group in the 2-, 3-, or 4-position, or an ester thereof,

$R^2$ represents a hydrogen atom or a lower alkyl group,

$R^3$ and $R^4$, which may be the same or different, each represents a hydrogen atom; an alkyl radical; or an unsubstituted or substituted aryl, arylalkyl or diarylalkyl radical; or

$R^3$ and $R^4$, together with the nitrogen atom to which they are attached, form a 5-, 6- or 7-membered saturated or unsaturated heterocyclic radical.

The invention also provides salts of compounds of the general formula (I), and more especially pharmaceutically acceptable salts thereof.

The compounds of the general formula (I) have, depending on the meaning of $R^2$, up to two asymmetric carbon atoms, marked with asterisks in the formula. These compounds may, therefore, exist in up to four

stereoisomeric forms. The present invention encompasses all stereoisomers of the compounds of the general formula (I) whether free from other isomers or admixed with other isomers in any proportion, and thus includes, for instance, racemic mixtures of enantiomers.

Alkyl groups may be straight or branched chain and may contain up to 12 carbon atoms. An alkyl radical represented by $R^3$ or $R^4$ or an alkyl moiety in an aralkyl or diarylalkyl radical represented by $R^3$ or $R^4$ is preferably a lower alkyl moiety.

When used herein in connection with alkyl radicals and moieties, or with alkanols, the term ''lower'' indicates that the radical, moiety or compound has from 1 to 6 carbon atoms, preferably 1 to 4 carbon atoms. An alkyl radical or moiety, including one in an alkanol compound, may be straight- or branched-chain, e.g. a methyl, ethyl, n-propyl, isopropyl, n-butyl, isobutyl or tert.-butyl group.

When used herein the term 'aryl' includes phenyl and naphthyl optionally substituted with up to five, preferably up to three, groups selected from halogen, $C_{1-6}$ alkyl, phenyl, $C_{1-6}$ alkoxy, halo($C_{1-6}$) alkyl, hydroxy, amino, nitro, carboxy, $C_{1-6}$ alkoxycarbonyl, $C_{1-6}$ alkoxycarbonyl-($C_{1-6}$)-alkyl $C_{1-6}$ alkylcarbonyloxy, or $C_{1-6}$ alkylcarbonyl groups.

A preferred aryl radical is phenyl, preferably unsubstituted phenyl.

Suitably, when $R^1$ represents a hydroxy group it may be derivatised as an ester, by for example, an arylalkyl carboxylic acid or a lower alkyl carboxylic acid. Suitable esters are in-vivo hydrolysable esters.

When $NR^3R^4$ forms a 5-, 6- or 7-membered saturated or unsaturated heterocyclic radical ring this may, if desired, contain a further hetero atom, suitably O, N or S. For example, $NR^3R^4$ represents a 1-pyrrolidinyl or 1-piperidyl group, or a 1-piperazinyl or a 4-morpholinyl radical.

Preferably, $R^3$ and $R^4$ each represents a hydrogen atom or a methyl group; more especially $R^3$ represents a hydrogen atom and $R^4$ represents a methyl group.

When used herein the term ''in-vivo hydrolysable ester'' relates to a pharmaceutically acceptable ester which readily breaks down in the human or non-human animal body to leave the free hydroxy group. Suitably in-vivo hydrolysable ester groups are those used conventionally in the art; they are preferably those provided by lower alkyl carboxylic acids.

Preferably, R represents a free hydroxy group; preferably $R^1$ represents a free hydroxy group.

Preferably $R^1$ is in the 4-position.

Compounds in which $R^2$ represents a hydrogen atom or a methyl group should especially be mentioned. Preferably $R^2$ represents a methyl group.

Thus, a preferred compound of the general formula (I) is one in which:

R represents a hydroxy group

$R^1$ represents a hydroxy group in the 4-position,

$R^2$ represents a hydrogen atom or a methyl group,

$R^3$ represents a hydrogen atom, and

$R^4$ represents a methyl group.

In a particularly preferred aspect the present invention provides a compound selected from:
N-methyl 4-[2-[3-(4-hydroxyphenoxy)-2-hydroxypropylamino]-2-methylethoxy]phenoxyacetamide; and

N-methyl 4-[2-[3-(4-hydroxyphenoxy)-2-hydroxypropylamino]ethoxy]phenoxyacetamide; or a pharmaceutically acceptable salt thereof.

The absolute configuration of any compound of the general formula (I) may be determined by conventional X-ray crystallographic techniques.

Preferably the asymmetric carbon atoms have the following configurations:

*: the S-configuration,

**: the R-configuration.

Compounds of the general formula (I) form acid addition salts.

Acid addition salts may be, for example, salts with inorganic acids such, for example, as hydrochloric acid, hydrobromic acid, orthophosphoric acid or sulphuric acid, or with organic acids such, for

example as methanesulphonic acid, toluenesulphonic acid, acetic acid, propionic acid, lactic acid, citric acid, fumaric acid, malic acid, succinic acid, salicylic acid or acetylsalicylic acid.

The invention also provides a process for the preparation of a compound of the general formula (I) or a pharmaceutically acceptable salt thereof, which comprises reacting a compound of the general formula (II):

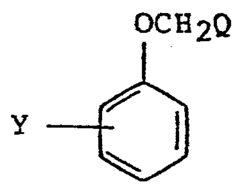

(II)

with a compound of the general formula (III):

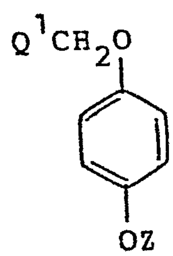

(III)

wherein

Y represents a hydroxy group or a protected hydroxy group,

Z represents the radical $CH_2CONR^3R^4$ or a radical convertible to $CH_2CONR^3R^4$,

Q represents a group of the formula (a) or (b):

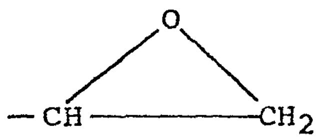 or 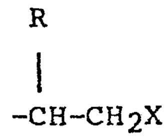

(a) (b)

in which

R is as defined in relation to formula (I) and

X represents a leaving group, and

$Q^1$ represents a group of the formula (A):

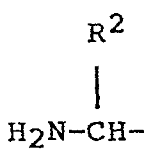 (A)

in which $R^2$ is as defined in relation to formula (I) or

Q represents a group of the formula (c):

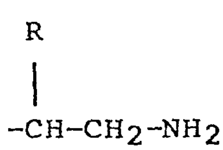 (c)

in which R has the meaning given above, and

$Q^1$ represents a group of the formula (B):

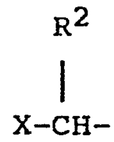

(B)

in which $R^2$ and X have the meanings given above, and, if required, converting any hydroxy protecting group represented by Y into a free hydroxy group and/or converting a group represented by Z into the radical $CH_2CONR^3R^4$, and, if desired, converting a resulting compound of the general formula (I) into another such compound and/or if desired, converting a salt into a free compound of formula (I), or a compound of formula (I) into a pharmaceutically acceptable salt thereof.

The present invention also provides a process for the preparation of a compound of the general formula (I) or a pharmaceutically acceptable salt thereof, which comprises reducing a compound of the general formula (IV):

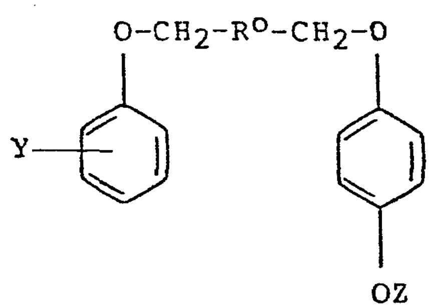

(IV)

in which

Y and Z have the meanings given above and $R^0$ represents a group of the formula

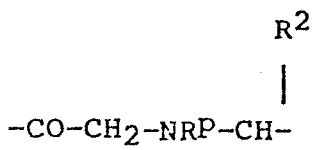

(i),

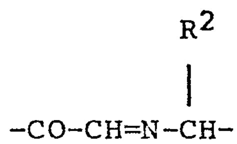

(ii),

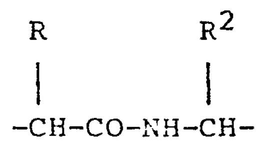

(iii),

or

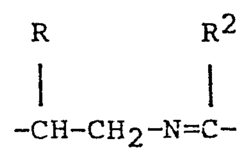

(iv)

in which $R^p$ is hydrogen or a protecting group, preferably a benzyl group, R and $R^2$ are as defined in relation to formula (I) provided that $R^0$ does not represent a group of the formula (iii) when Z represents a group of the formula $CH_2CONR^3R^4$, and, if required, removing any protecting group represented by $R^p$, converting any protected hydroxy group represented by Y into a free hydroxy group and/or converting a group represented by Z into the radical $CH_2CONR^3R^4$, and, if desired, converting a resulting compound of the general formula (I) into another such compound and/or if desired, converting a salt into a free compound of formula (I), or a compound of formula

(I) into a pharmaceutically acceptable salt thereof.

The present invention also provides a compound of the general formula (IV) shown above in which Y, Z and $R^0$ have the meanings given above, and salts thereof.

These starting materials of the general formula (IV) may be prepared, for example, by reacting a compound of the general formulae (II) and (III) in which:

Y and Z have the meanings given above,
Q represents a group of the formula (d), (e) or (f):

| (d) | (e) | | (f) |
|---|---|---|---|
| $-CO-CH_2X$, | $-CO-CHO$ | or | R<br>\|<br>$-CH-COOH$ |

in which R is as defined in relation to formula (I) and X is as defined in relation to formula (II), provided that Q does not represent a group of the formula (f) when Z represents a group of the formula $CH_2CONR^3R^4$,

and

$Q^1$ represents a group of the formula (A):

$R^2$
|
$NH_2-CH-$ (A)

in which $R^2$ has the meaning given above,

or

Q represents a group of the formula (c):

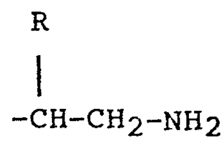

(c)

in which R has the meaning given above, and $Q^1$ represents a group of the formula (J):

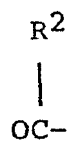

(J)

in which $R^2$ has the meaning given above.

The invention also provides a process for the preparation of a compound of the general formula (I) or a pharmaceutically acceptable salt thereof, which comprises treating a compound of the general formula (V):

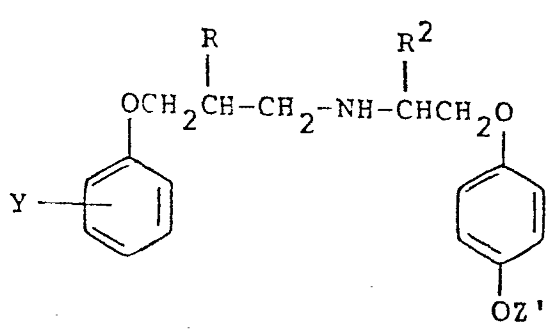

V

in which

R and $R^2$ are as defined in relation to formula (I), and Y is as defined in relation to formula (II),

and Z' represents a radical convertible to $CH_2CONR^3R^4$, wherein $R^3$ and $R^4$ are as defined in relation to formula (I), or, when Y represents a protected hydroxy group, the radical $CH_2CONR^3R^4$, or a salt thereof, to convert Y into a free hydroxy group and/or to convert a group represented by Z' into the radical $CH_2CONR^3R^4$, and, if desired, converting a resulting compound of the general formula (I) into another such compound, and/or, if desired, converting a salt into a free compound of formula (I), or a compound of formula (I) into a pharmaceutically acceptble salt thereof or a salt into a free compound.

Preferably in the above reactions to prepare a compound of the general formula (I) Y represents a protected hydroxy group. Suitable hydroxy protecting groups are those used routinely in the art, preferably the hydroxy group is protected by etherification.

A hydroxy group protected by etherification and represented by Y may be converted into a free hydroxy group by methods known per se. For example, Y may represent an unsubstituted or substituted benzyloxy group, which is converted by hydrogenolysis into a free hydroxy group. The hydrogenolysis reaction may be carried out, for example in the presence of a palladium-on-carbon catalyst, in a solvent, for example a mixture of ethyl acetate and methanol.

Z and Z' may represent the radical $-CH_2CONR^3R^4$ or a group convertible thereto, for example by reaction of an ester with an amine of the general formula $HNR^3R^4$ or by hydrolysis of a nitrile or by alkylation of a hydroxy group.

Thus, for example Z and Z' may represent $CH_2COOR^5$ where $R^5$ represents a lower alkyl radical, especially a methyl or ethyl group, or the group $-CH_2CN$. Conversion of a $CH_2COOR^5$ radical into $CH_2CONR^3R^4$ may be carried out, for example, with methylamine in, for example, a lower alkanol, and hydrolysis of $-CH_2CN$ to form a $-CH_2CONH_2$ group by, for example, controlled acid hydrolysis; or -OZ or-$OZ^1$ may represent a free hydroxy group which is alkylated with for example a compound $XCH_2COR^3R^4$ wherein X, $R^3$ and $R^4$ are as defined above.

A resulting compound of the general formula (I) may, if desired, be converted into another compound of the general formula (I), for example by carrying out one or more of the following reactions where appropriate in any desired order:

(a) converting a substituted amide into an unsubstituted amide or vice-versa,

(b) converting a hydroxy group into an ester thereof,

(c) converting an esterified hydroxy group into a free hydroxy group.

The present invention also provides a compound of the general formula (V) shown above in which R and $R^2$ are as defined in relation to formula (I) and Y and Z' are as defined in relation to formula (V) and salts thereof.

Compounds of the general formula (V) may be prepared, for example, by reaction of compounds of the general formulae (II) and (III) wherein

Y has the meaning given above,

Z is Z′ as defined in relation to formula (V) and

Q represents a group of the formula (a) or (b) and $Q^1$ represents a group of the formula (A); or

Q represents a group of the formula (c) and

$Q^1$ represents a group of the formula (B);

or by reduction of a compound of the general formula (IV) in which Y and $R^o$ are as defined in relation to formula (IV) and Z is Z′as defined in relation to formula (V).

More particularly, compounds of the general formula (V) may be prepared by reaction of a compound of the general formula (IIIA):

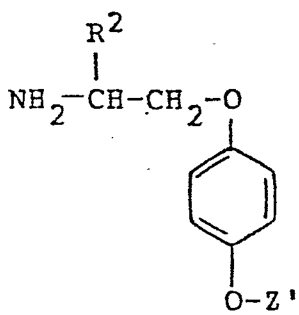

IIIA

in which $R^2$ and Z' have the meanings given above with a compound of the general formula (IIa):

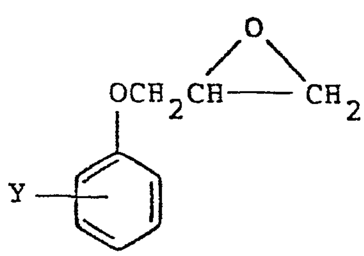

IIa

in which Y is as defined in relation to formula (II).

Compounds of the general formula (V) may also be prepared by reacting a compound of the general formula (IIIA) with a compound of the general formula (IIb)

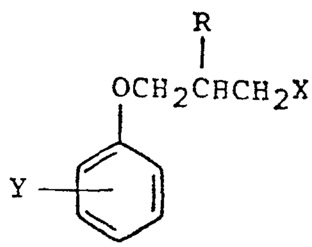

IIb

in which R, is as defined in relation to formula (I) Y and X are as defined in relation to formula (II).

Compounds of the general formula (V) may furthermore be prepared by reacting a compound of the general formula (IIc):

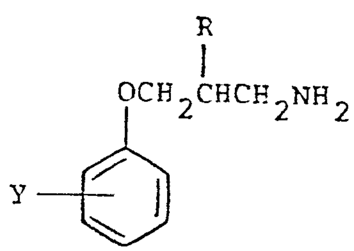

IIc

in which R and Y are as defined in relation to formula (IIb) with a compound of the general formula (IIIb)

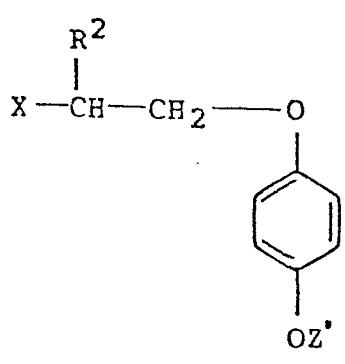

IIIB

in which $R^2$, Z' and X have the meanings given above.

Compounds of the general formula (V) may also be prepared by reacting a compound of the general formula (IIIA) with a compound of the general formula (IId):

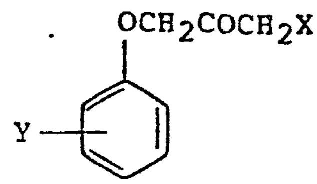

IId

in which X and Y are as defined in relation to formula (IIb) followed by reduction of the ketone group.

Compounds of the general formula (V) may further be prepared by reacting a compound of the general formula (IIIA) with a compound of the general formula (IIe):

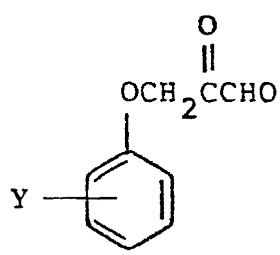

IIe

in which Y is as defined in relation to formula (IIb), followed by reduction of the ketone and imine groups.

Compounds of the general formula (V) may further be prepared by reacting a compound of the general formula (IIIA), in which $R^2$ is as defined in relation to formula (I) and Z' is as defined in relation to formula (V), provided that Z′ does not represent a group of the formula $CH_2CONR^3R^4$, with a compound of the general formula (IIf):

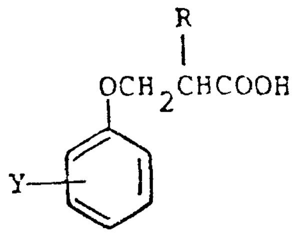

IIf

in which R is as defined in relation to formula (I) and Y is as defined in relation to formula (II), followed by reduction of the carbonyl group of the resulting amide.

In another method of preparation of starting materials of the general formula (V), a compound of the general formula (IIc) in which R and Y are as defined in relation to formula (IIf), is reacted with a compound of the general formula (IIIc)

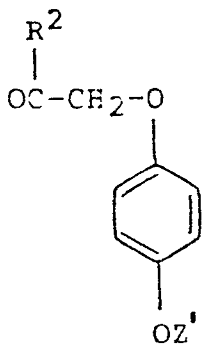

IIIC

in which $R^2$ is as defined in relation to formula (I) and Z' is as defined in relation to formula (V), followed by reduction of the imine group.

A leaving group X is any group that will, under the reaction conditions, cleave from the starting material, thus promoting reaction at a specified site. Suitable examples of such groups are halogen atoms, mesyloxy groups and tosyloxy groups. Preferably in formula (b) of compound (II) (or compound IIb) or in formula (B) of compound (III) or (compound IIIB) X represents a mesyloxy or tosyloxy group or a bromine atom, and in formula (d) of compound (II) (or compound IId) X represents a bromine atom.

Compounds of formulae (II) and (III) are either known compounds or can be prepared from known compounds by known processes or processes analogous to known processes.

The reaction of compounds of the general formulae (II) and (III) in which Q and $Q^1$ have formulae (a) and (A) respectively (or reaction between compounds of formula IIa and IIIA) is advantageously carried out in a protic solvent, e.g. an alkanol, especially a lower alkanol having at least 2 carbon atoms, at reflux, preferably in ethanol.

Reaction of compounds of the general formulae (II) and (III) in which Q and $Q^1$ have formulae (b) and (A) or (c) and (D) respectively (or reaction between compounds of formulae: IIb and IIIA, or IIc and IIIB) is advantageously carried out in dimethyl sulphoxide, for example at a temperature in the range of from 30 to 80°C, e.g. substantially 50°C, and advantageously for a period of time of 1 to 4 days, e.g. about 3 days.

The reaction of the compounds of the general formulae (II) and (III) in which Q and $Q^1$ have formulae (d) and (A) respectively (or reaction between compounds of of formulae IId and IIIA) is advantageously carried out in butanone or acetonitrile at reflux, if desired in the presence of a base.

The reaction of compounds of the general formula (II) and (III) in which Q and $Q^1$ have formulae (e) and (A) or (c) and (J) respectively (or reaction between compounds of formulae IIe and IIIA and IIc and IIIC) is preferably carried out in benzene using a Dean and Stark apparatus, more especially at reflux.

The reaction of compounds of the general formulae (II) and (III) in which Q and $Q^1$ have formulae (f) and (A) respectively (or reaction between compounds of formulae IIf and IIIA) is preferably carried out in the presence of dicyclohexyl carbodiimide or other suitable condensing agent.

The reduction of a compound of the general formula (IV) may be carried out, for example,

when $R^o$ represents a group of the formula (i) and (ii) with sodium borohydride;

when $R^o$ represents a group of the formula (iii) with a borane reducing agent, for example borane methyl sulphide complex, and

when $R^o$ represents a group of the formula (iv) with sodium borohydride or sodium cyanoborohydride, or catalytically, for example by hydrogen in the presence of platinum or palladium.

The reductions with sodium cyanoborohydride and sodium borohydride are preferably performed in a lower alkanol, e.g. methanol.

A compound of the general formula (V) may, if desired, be converted into another compound of the general formula (V) or a compound of the general formula (IV) into another compound of the general formula (IV); by a method known *per se*. For example, an ester may be converted into an alcohol, or an alcohol into an ester thereof, or a substituted amide into an unsubstituted amide, or other group represented by Z or Z' may be converted into another such group.

0171760

In a preferred method for preparing a compound of the general formula (I), a compound of the general formula (IIc), preferably a compound of the formula (IIc'):

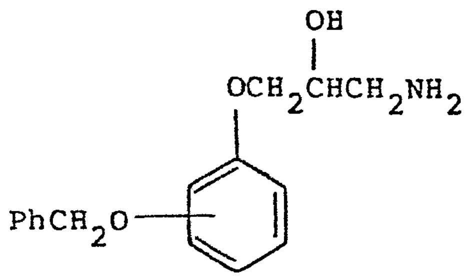

IIc'

in which Ph represents a phenyl group, is reacted with a compound of the general formula (IIIC), preferably a compound of the general formula (IIIC'):

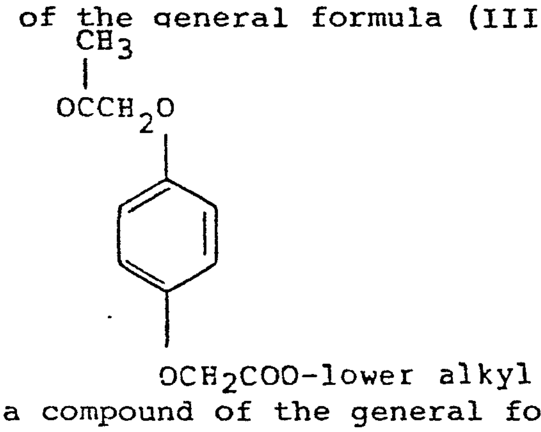

IIIC'

to form a compound of the general formula (IV) in which $R^o$ represents a group of the general formula (iv) which is then reduced with sodium cyanoborohydride, preferably in methanol, to give a compound of the general formula (V), in which Z' represents a group $CH_2COO$-lower alkyl; this is then reacted with methylamine, preferably in ethanol, to give a compound of the general formula (V) in which Z' represents the group $CH_2CONHCH_3$ and this is then subjected to hydrogenolysis, preferably with the use of a palladium-on-carbon catalyst, to prepare a compound of the general formula (I).

The salts of compounds of the general formula (I) may be produced, for example, by treating a compound of general formula (I) with the appropriate acid.

Compounds of the general formula (I) and salts thereof, produced by the above processes, may be recovered by conventional methods.

Compounds of the general formula (I) may be separated into diastereoisomeric pairs of enantiomers by, for example, fractional crystallisation from a suitable solvent, for example methanol or ethyl acetate or a mixture thereof. The pair of enantiomers thus obtained may be separated into individual stereoisomers by conventional means, for example by the use of an optically active acid as a resolving agent.

Suitable optically active acids which maybe used as resolving agents are described in 'Topics in Stereochemistry', Vol. 6, Wiley Interscience, 1971, Allinger, N.L. and Eliel, W.L. Eds.

Alternatively, any enantiomer of a compound of the general formula (I) may be obtained by stereospecific synthesis using optically pure starting materials of known configuration.

As previously indicated, the compounds of the present invention have valuable pharmacological properties.

The present invention also provides a compound of the general formula (I) or a pharmaceutically acceptable salt thereof for use as an active therapeutic substance.

The present invention further provides a compound of the general formula (I), or a pharmaceutically acceptable salt thereof, for use in the manufacture of a medicament for the treatment of congestive heart failure in the human or animal body.

The present invention further provides a compound of the general formula (I), or a pharmaceutically

acceptable salt thereof, for use in the treatment of congestive heart failure in the human or non-human animal body.

The compounds may be administered per se or, preferably, as a pharmaceutical composition also comprising a pharmaceutically acceptable carrier.

Accordingly, the present invention also provides a pharmaceutical composition comprising a compound of the general formula (I) or a pharmaceutically acceptable salt thereof, in admixture or conjunction with a pharmaceutically acceptable carrier therefor.

As used herein, the terms ''pharmaceutical composition'' and ''pharmaceutically acceptable'' embrace compositions and ingredients for both human and veterinary use.

The composition may, if desired, be in the form of a pack accompanied by written or printed instructions for use.

Usually the pharmaceutical compositions of the present invention will be adapted for oral administration, although compositions for administration by other routes, such as by injection, are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or other conventinal adjuvant.

Typical carriers include, for example, microcrystalline cellulose, starch, sodium starch glycollate, polyvinylpyrrolidone, polyvinylpolypyrrolidone, magnesium stearate, sodium lauryl sulphate or sucrose.

Most suitably the composition will be formulated in unit dose form. Such unit dose will normally contain an amount of the active ingredient in the range of from 0.1 to 500 mg, more usually 0.1 to 250 mg, and more especially 0.1 to 100 mg.

The present invention further provides a method for treating congestive heart failure in a human or non-human animal, which comprises administering an effective, non-toxic, amount of a compound of the general formula (I) or pharmaceutically acceptable salt thereof to a human or non-human animal.

Conveniently, the active ingredient may be administered as a pharmaceutical composition hereinbefore defined, and this forms a particular aspect of the present invention.

In treating humans with congestive heart failure the compound of the general formula (I) or pharmaceutically acceptable salt thereof may be taken in doses, such as those described above, one to six times a day in a manner such that the total daily dose for a 70 kg adult will generally be in the range of from about 0.1 to 1000 mg, and more usually about 1 to 500 mg. In general a total daily dosage of from about $1.4 \times 10^{-3}$ mg/kg to 15.0 mg/kg, and more usually from about $1.4 \times 10^{-2}$ mg/kg to 7.50 mg/kg is suitable.

No toxicological effects are indicated when the compounds of the invention or pharmaceutically acceptable salts thereof are administered in any of the above mentioned dosage ranges.

The following Example illustrates the invention but do not limit it in any way.

Example 1

N-Methyl 4-[2-[3-(4-hydroxyphenoxy)-2-hydroxypropylamino]-2-methylethoxy]phenoxyacetamide

3-[4-Benzyloxyphenoxy]-2-hydroxypropanamine (2.73 g), ethyl 4-acetonyloxyphenoxyacetate (2.52 g) and sodium cyanoborohydride (0.63 g) were allowed to stand in methanol at ambient temperature for 18h. The solvent was evaporated, the residue partitioned between water and ethyl acetate, and the organic extracts dried ($MgSO_4$). Evaporation of the solvent gave an oil which was chromatographed on kieselgel. Elution with methanol/ammonia/chloroform (5 : 0.5 : 94.5) gave ethyl 4-[2-[3-(4-benzyloxyphenoxy)-2-hydroxypropylamino]-2-methylethoxy]phenoxyacetate (0.75 g). This was dissolved in ethanol and treated with ethanolic methylamine. Removal of the solvent gave N-methyl 4-[2-[3-(4-benzyloxyphenoxy)-2-hydroxypropylamino]-2-methylethoxy]phenoxyacetamide (0.7 g) which was hydrogenated at ambient temperature and pressure in ethyl acetate/methanol using 10 % Pd/C as catalyst. The catalyst was filtered off and the solvent evaporated to give N-methyl 4-[2-[3-(4-hydroxyphenoxy)-2-hydroxypropylamino]-2-methylethoxy]phenoxyacetamide (0.35 g) as a 1:1 mixture of diastereoisomers, m.p. 105-112° (ethyl acetate/methanol).

$^1H$ nmr ($d_6$-DMSO), ppm:

1.05 (3H,d), 2.65 (3H, doublet, collapses to singlet with $D_2O$ + 2H,m), 2.9 (1H,q), 3.7-3.9 (5H,m), 4.32 (2H,s), 1.5-4.5 (approx. 2H, very broad, disappears with $D_2O$), 6.68 (4H,s), 6.88 (4H,s), 7.9 (1H, broad, disappears with $D_2O$), 8.85 (1H, broad, disappears with $D_2O$).

EXAMPLE 2



N-Methyl 4-[2-[3-(4-hydroxyphenoxy)-2-hydroxypropylamino]-ethoxy]phenoxyacetamide

3-(4-Benzyloxyphenoxy)-2-hydroxypropanamine (2.75 g), N-methyl 4-(2-hydroxyethoxy)phenoxyacetamide-4-toluenesulphonate (3.80 g) and triethylamine (4.1 ml) were dissolved in dimethylsulphoxide and heated at 50-60° for 3 days. The cooled reaction mixture was then poured into water and filtered. The residue was dissolved in ethyl acetate, dried (magnesium sulphate), filtered, evaporated and recrystallized from ethyl acetate to give N-methyl 4-[2-[3-(4-benzyloxyphenoxy)-2-hydroxypropylamino]ethoxy]phenoxyacetamide (0.8 g) which was hydrogenated at ambient temperature and pressure in glacial acetic acid using 10% palladium on carbon as catalyst. After filtration of the catalyst and evaporation of the solvent *in vacuo* the residue was treated with sodium hydrogen carbonate solution (50 ml) and extracted with ethyl acetate (3x50 ml). The combined extracts were dried (magnesium sulphate), filtered and evaporated under reduced pressure. The residue was purified by column chromatography on silica gel using methanol-chloroform (5:95) as eluent to give N-methyl 4-[2-[3-(4-hydroxyphenoxy)-2-hydroxypropylamino]ethoxy]-phenoxyacetamide, m.p. 128-130° (ethyl acetate/methanol).

$^1H$ nmr (DMSO-$d_6$), ppm

2.65 (3H, doublet, collapses to singlet on $D_2O$); 2.85 (2H, t); 2.2-3.6 (2H, very broad, disappears on $D_2O$); 3.6-4.1 (7H,m); 4.4 (2H,s); 6.7 (4H,m); 6.9 (4H,s); 8.0 (1H, broad, disappears on $D_2O$); 8.6-9.3 (1H, very broad, disappears on $D_2O$).

N-Methyl 4-(2-hydroxyethoxy)phenoxyacetamide-4-toluenesulphonate

4-Toluenesulphonyl chloride (10.4 g) was added to a solution of N-methyl 4-(2-hydroxyethoxy)phenoxyacetamide (8.2 g) in dry pyridine at 0-5$^{O}$. The mixture was kept overnight at 0-5$^{O}$, the solvent evaporated under reduced pressure and the residue dissolved in ethyl acetate. The organic solution was washed with 2N hydrochloric acid (1x50 ml), water (2x50 ml) and brine (1x50 ml) and the organic layer was dried ($MgSO_4$), filtered and evaporated to give N-methyl 4-(2-hydroxyethoxy)-phenoxyacetamide-4-toluenesulphonate.

$^{1}H$ nmr (DMSO-$d_6$), ppm

2.4 (3H,s); 2.7 (3H,d); 4.2 (4H,dd); 4.4 (2H,s); 6.8 (4H,s); 7.45 (2H,d); 7.8 (2H,d); 8.0 (1H,b).

EXAMPLE X2

0171760

N-Methyl 4-(2-hydroxyethoxy)phenoxyacetamide

A solution of methyl 4-(2-hydroxyethoxy)phenoxyacetate (10 g) in methanol was treated with excess methylamine (33% solution in ethanol) at room temperature. The solution was allowed to stand for 3 hours. Removal of the solvent under reduced pressure gave N-methyl 4-(2-hydroxyethoxy)-phenoxyacetamide.

$^1H$ nmr (DMSO-$d_6$), ppm

2.6 (3H,d); 3.7 (4H,m); 4.3 (2H,s); 4.75 (1H,b); 6.8 (4H,s); 7.9 (1H,b).

EXAMPLE X3

Methyl 4-(2-hydroxyethoxy)phenoxyacetate

Methyl bromoacetate (26.5 g) was added to a mixture of 4-(2-hydroxyethoxy)phenol (17.8 g), potassium carbonate (20 g), and potassium iodide (0.5 g) in butan-2-one at room temperature. The mixture was heated under reflux for 4 hours, cooled and filtered. The filtrate was evaporated under reduced pressure, the residue taken up in ethyl acetate, washed with water (2x50 ml) and the organic layer dried ($MgSO_4$). Evaporation of the solvent gave a pale solid which was purified by column chromatography on silica gel using chloroform as eluent to give methyl 4-(2-hydroxyethoxy)phenoxyacetate

$^1H$ nmr ($CDCl_3$), ppm

3.1 (1H,m); 3.7 (3H,s); 3.85 (4H,m); 4.5 (2H,s); 6.75 (4H, s).

## DEMONSTRATION OF EFFECTIVENESS OF COMPOUNDS

### a) Activity of Compounds on Isolated Rat Atria

Sprague-Dawley male rats (300-400 g) are killed and the right and left atria dissected out independently and mounted on a combined tissue holder/electrode. The tissues are then immersed in a glass bath containing Krebs solution maintained at 32°C. The rate of the spontaneously beating right atrium (via an isometric transducer and a ratemeter) and the tension of the electrically paced left atrium (via an isometric transducer) are recorded on a M19 chart recorder.

After an initial stabilization period, the tissues are exposed to a maximal concentration of isoprenaline ($10^{-7}$M). The tissues are then washed several times until rate and tension return to baseline level. A dose-response curve to the test compound is then carried out.

Responses to each concentration of test compound are expressed as a percentage of the maximal responses to isoprenaline. Results are given in the form of (a) intrinsic activity (maximal effect of test compound with respect to isoprenaline maximum, i.e. isoprenaline intrinsic activity = 1) and (b) $EC_{50}$ (the molar concentration at which the test compound produces 50% of its own maximum response).

| Compound of Example No. | Rate | | Tension | |
|---|---|---|---|---|
| | Intrinsic activity | $EC_{50}$ | Intrinsic activity | $EC_{50}$ |
| 1 | 0.94 | $1x10^{-8}$ | 0.88 | $2.5x10^{-8}$ |
| 2 | 0.77 | $6.8x10^{-8}$ | 0.61 | $1.5x10^{-7}$ |

b) Assessment of Inotropic Activity in Conscious Cats

Male 3-4.5 kg cats, bred in-house, are implanted with a polythene cannula in the left ventricle which is exteriorised at the back of the neck by means of a small teflon valve. Recordings are made via a pressure transducer attached by means of a length of polyethylene tubing to the cat neck valve. The amplified left ventricular pressure (LVP) signal is displayed on a chart recorder and, from this, signal measurements of heart rate (HR) are obtained via a ratemeter, and rate of change (dp/dt) of left ventricular pressure (LVP) is obtained via a differentiator.

During an initial 1 hour stabilisation period, steady baseline recordings are obtained. The test compound, contained in a gelatin capsule, is then administered orally. Recording is normally carried out for 6 hours post dosing.

The maximal percentage change in dp/dt over initial baseline level is used as the index of inotropic activity. Chronotropic activity is taken as the maximal percentage change in HR over initial baseline level.

| Compound of Example No. | Dose mg/kg p.o. | Peak dp/dt | Peak HR |
|---|---|---|---|
| 1 | 0.2 | 22 | 0 |

0171760

CLAIMS

A and C

1. A compound of the general formula (I)

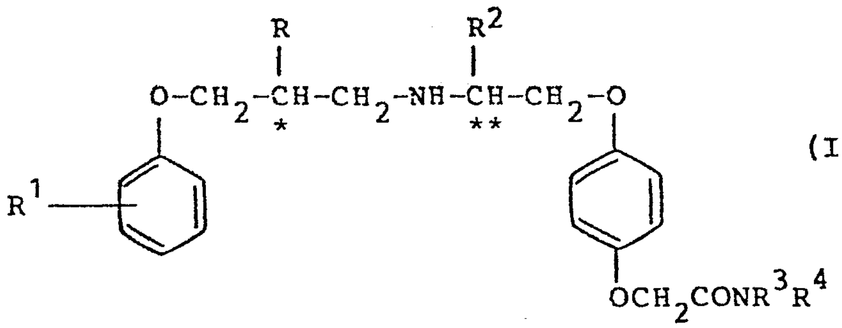

(I)

or a pharmaceutically acceptable salt thereof, in which

R represents a hydroxy group or an in vivo hydrolysable ester thereof,

$R^1$ represents a hydroxy group in the 2-, 3-, or 4-position, or an ester thereof,

$R^2$ represents a hydrogen atom or a lower alkyl group,

$R^3$ and $R^4$, which may be the same or different, each represents a hydrogen atom; an alkyl radical; or an unsubstituted or substituted aryl, arylalkyl or diarylalkyl radical; or

$R^3$ and $R^4$, together with the nitrogen atom to which they are attached, form a 5-, 6- or 7-membered saturated or unsaturated heterocyclic radical.

2. A compound as claimed in claim 1, wherein $R^3$ represents a hydrogen atom and $R^4$ represents a methyl group.

3. A compound as claimed in claim 1 or claim 2 wherein R and $R^1$ each represents a free hydroxyl group.

4. A compound as claimed in any one of claims 1 to 3, wherein $R^1$ is in the 4-position.

5. A compound as claimed in claim 1, wherein:

R represents a hydroxy group,
$R^1$ represents a hydroxy group in the 4-position,
$R^2$ represents a hydrogen atom or a methyl group
$R^3$ represents a hydrogen atom, and
$R^4$ represents a methyl group.

6. A compound as claimed in claim 1, selected from the group consisting of:

N-Methyl 4-[2-[3-(4-hydroxyphenoxy) -2-hydroxypropylamino]-2-methylethoxy]phenoxyacetamide; and
N-methyl 4-[2-[3-(4-hydroxyphenoxy)-2-hydroxypropylamino]ethoxy]phenoxyacetamide; or a pharmaceutically acceptable salt thereof.

7. A process for the preparation of a compound of the general formula (I) or a pharmaceutically acceptable salt thereof, which comprises:

A. reacting a compound of the general formula (II):

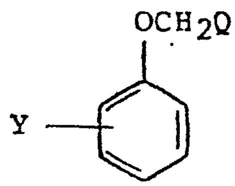

(II)

with a compound of the general formula (III):

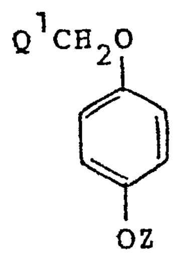

(III)

wherein

Y represents a hydroxy group or a protected hydroxy group,

Z represents the radical $CH_2CONR^3R^4$ or a radical convertible to $CH_2CONR^3R^4$,

Q represents a group of the formula (a) or (b):

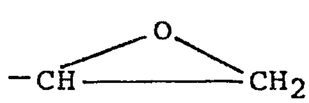

or

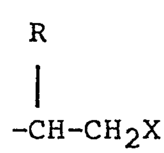

(a) (b)

in which

R is as defined in relation to formula (I) and
X represents a leaving group, and
$Q^1$ represents a group of formula (A):

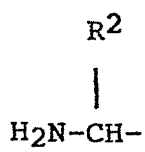

(A)

in which $R^2$ is as defined in relation to formula (I) or
Q represents a group of the formula (c):

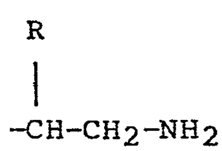

(c)

in which R has the meaning given above, and
$Q^1$ represents a group of the formula (B):

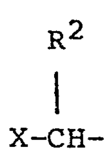

(B)

in which $R^2$ and X have the meanings given above, and, if required, converting any protected hydroxy group represented by Y into a free hydroxy group and/or converting a group represented by Z into the radical $CH_2CONR^3R^4$; or

B. reducing a compound of the general formula )IV):

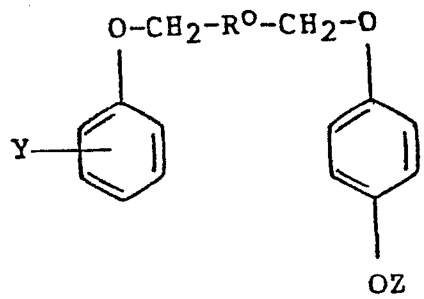

(IV)

in which

Y and Z have the meanings given above and $R^0$ represents a group of the formula

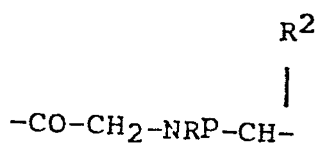

(i),

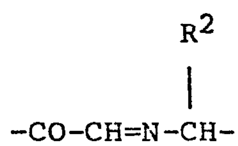

(ii),

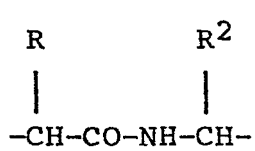

(iii),

or

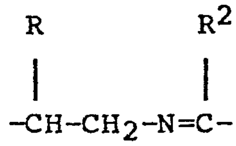 (iv)

in which $R^P$ is hydrogen or a protecting group, preferably a benzyl group, R and $R^2$ are as defined in relation to formula (I) provided that $R^0$ does not represent a group of the formula (iii) when Z represents a group of the formula $CH_2CONR^3R^4$, and, if required, removing any protecting group represented by $R^P$, converting any protected hydroxy group represented by Y into a free hydroxy group and/or converting a group represented by Z into the radical $CH_2CONR^3R^4$; or

C. treating a compound of the general formula (V):

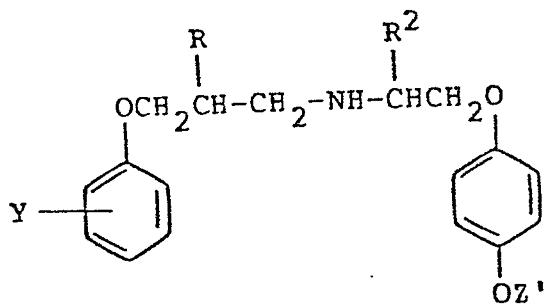 (V)

in which

R and $R^2$ are as defined in relation to formula (I), and Y is as defined in relation to formula (II),

and Z' represents a radical convertible to $CH_2CONR^3R^4$, wherein $R^3$ and $R^4$ are as defined in relation to formula (I), or, when Y represents a protected hydroxy group, the radical $CH_2CONR^3R^4$, or a salt thereof, to convert Y into a free hydroxy group and/or to convert a group represented by Z' into the radical $CH_2CONR^3R^4$; or

D. reacting a compound of the formula (IIc′):

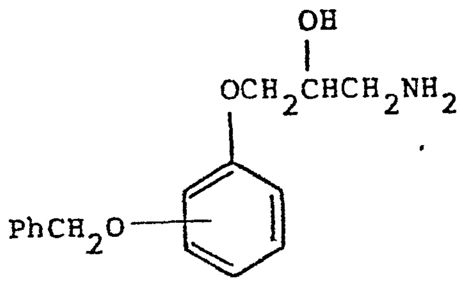

(IIc')

in which Ph represents a phenyl group, with a compound of the general formula (IIIC), preferably a compound of the general formula (IIIc′):

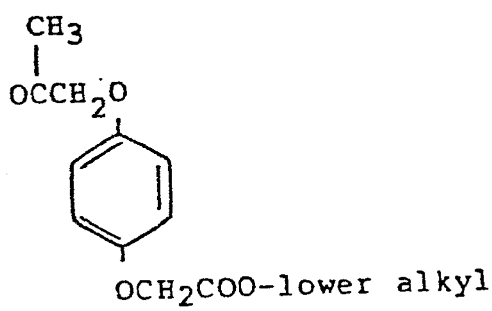

(IIIC')

to form a compound of the general formula (IV) in which $R^o$ represents a group of the general formula (iv) which is then reduced with sodium cyanoborohydride, preferably in methanol, to give a compound of the general formula (V), in which Z' represents a group $CH_2COO$-lower alkyl; this is then reacted with methylamine, preferably in ethanol, to give a compound of the general formula (V) in which Z' represents the group $CH_2CONHCH_3$ and this is then subjected to hydrogenolysis, preferably with the use of a palladium-on-carbon catalyst, to prepare a compound of the general formula (I); and, if desired, converting a resulting compound of the general formula (I) into another such compound, and/or, if desired, converting a salt into a free compound of formula (I), or a compound of formula (I) into a pharmaceutically acceptable salt thereof.

8. A compound of formula (I) or a pharmaceutically acceptable salt thereof, for use as an active therapeutic substance.

9. A compound of the general formula (I), or a pharmaceutically acceptable salt thereof, for use in the treatment of congestive heart failure in the human or non-human animal body.

10. A pharmaceutical composition comprising a compound of the general formula (I) or a pharmaceutically acceptable salt thereof, in admixture or conjunction with a pharmaceutically acceptable carrier therefore.

11. A compound of the general formula(I), or a pharmaceutically acceptable salt thereof, for use in the manufacture of a medicament for the treatment of congestive heart failure in the human or non-human animal body.